# EUROPEAN PATENT SPECIFICATION

(11) **EP 3 467 507 B1**
(45) Date of publication and mention of the grant of the patent: **23.02.2022**
(21) Application number: 17802965.8
(22) Date of filing: 17.03.2017
(51) Int. Cl.: G01N 33/68, G01N 33/50

(54) **METHOD FOR TESTING NK CELL ACTIVITY USING SYNERGISTIC ACTIVITY OF RECEPTOR, AND METHOD FOR DIAGNOSING DISEASE ASSOCIATED WITH NK CELL ACTIVITY USING SAME**
VERFAHREN ZUM TESTEN DER NK-ZELLAKTIVITÄT MIT SYNERGISTISCHER REZEPTORWIRKUNG UND VERFAHREN ZUR DIAGNOSE VON KRANKHEITEN IM ZUSAMMENHANG MIT DER NK-ZELLAKTIVITÄT DAMIT
PROCÉDÉ D'ANALYSE DE L'ACTIVITÉ DE CELLULES TUEUSES NATURELLES À L'AIDE DE L'ACTIVITÉ SYNERGIQUE D'UN RÉCEPTEUR, ET PROCÉDÉ DE DIAGNOSTIC DE MALADIE ASSOCIÉE À L'ACTIVITÉ DES CELLULES TUEUSES NATURELLES UTILISANT LEDIT PROCÉDÉ D'ANALYSE

(30) Priority: 24.05.2016 KR 20160063735; 16.03.2017 KR 20170033207
(43) Date of publication of application: 10.04.2019
(73) Proprietor: University Of Ulsan Foundation For Industry Cooperation, Ulsan 44610 (KR); The Asan Foundation, Seoul 05505 (KR)
(72) Inventor: KIM, Hun Sik, Seoul 05118 (KR); MYUNG, Seung Jae, Seoul 06215 (KR); KIM, Song Cheol, Seoul 05507 (KR)
(74) Representative: Loyer & Abello
(86) International application number: PCT/KR2017/002870
(87) International publication number: WO 2017/204446

(56) References cited:
- KR-A- 20130 032 410
- US-A1- 2012 258 476
- US-A1- 2014 017 713
- AL-HUBESHY Z B ET AL: "A rapid method for assessment of natural killer cell function after multiple receptor crosslinking", JOURNAL OF IMMUNOLOGICAL METHODS, ELSEVIER SCIENCE PUBLISHERS B.V.,AMSTERDAM, NL, vol. 366, no. 1, 20 January 2011 (2011-01-20), pages 52-59, XP028172028, ISSN: 0022-1759, DOI: 10.1016/J.JIM.2011.01.007 [retrieved on 2011-01-28]
- Yenan T Bryceson ET AL: "Synergy among receptors on resting NK cells for the activation of natural cytotoxicity and cytokine secretion", Blood, 1 January 2006 (2006-01-01), pages 159-166, XP055548706, DOI: 10.1182/blood-2005-04- Retrieved from the Internet: URL:http://www.bloodjournal.org/content/bl oodjournal/107/1/159.full.pdf [retrieved on 2019-01-29]
- Y. T. BRYCESON ET AL: "Minimal requirement for induction of natural cytotoxicity and intersection of activation signals by inhibitory receptors", BLOOD, vol. 114, no. 13, 24 September 2009 (2009-09-24), pages 2657-2666, XP055548719, ISSN: 0006-4971, DOI: 10.1182/blood-2009-01-201632
- KIM et al.: "Synergistic Signals for Natural Cytotoxicity Are Required to Overcome Inhibition by c-Cbl Ubiquitin Ligase", Immunity, vol. 32, 2010, pages 175-186, XP055558563,
- KWON et al.: "Signaling for Synergistic Activation of Natural Killer Cells", Immune Network, vol. 12, no. 6, 2012, pages 240-246, XP055543960,
- LONG et al.: "Controlling Natural Killer Cell Responses: Integration of Signals for Activation and Inhibition", Annual Review of Immunology, vol. 31, 2013, pages 227-258, XP055558564,

## Description

### TECHNICAL FIELD

The present disclosure relates to a method of diagnosing hemophagocytic lymphohistiocytosis or cancer by using the synergic activation level of distinguishable NK factors NKG2D and 2B4(CD244).

### BACKGROUND ART

Natural killer cells (NK cells) are a type of cytotoxic lymphocytes important for innate and adaptive immune systems. NK cells respond to virus-infected cells or cancer cells, and typically detect abnormalities in the major histocompatibility complex (MHC) on cell surfaces. These NK cells secrete perforin to form pores in cell membranes of infected cells or cancer cells, and exert cytotoxicity that causes these cells to be killed by granzyme secreted by such NK cells. Patients with B cell lymphoma and many cancers are found to have defects in the number of NK cells or anticancer activity, and NK cell dysfunction is known to be closely related to the development of cancer (Annu. Rev. Immunol. 2013; 31:227-258). The documents Yenan T Bryceson ET Al "Synergy among receptors on resting NK cells for the activation of natural cytotoxicity and cytokine secretion" (01/01/2006) and Y. T. Bryceson ET AL "Minimal requirement for induction of natural cytotoxicity and intersection of activation signals by inhibitory receptors" (24/09/2009) concern assessment of NK activity.

Various activating and inhibitory receptors are involved in NK cell activity. Receptors considered dominant include those associated with immunoreceptor tyrosine-based activation motif (ITAM)-bearing signaling molecules, such as Nkp46 (the FcR γ chain or the TCR ζ chain), and the receptor NKG2D associated with the signaling molecule DAP10 (EMBO J. 2004; 23:255.-9, Annu. Rev. Immunol. 2005; 23:225-74). Receptors that are considered costimulatory include members of the signaling lymphocytic activation molecule (SLAM) family, such as 2B4 (CD244), as well as various receptors such as DNAM-1 (CD226), CD2, and NKp80 (product of the *KLRF1* gene).

ITAM-bearing molecules contribute to signal transduction by many different activation receptors on NK cells, and among natural cytotoxicity receptors (NCRs), NKp46 and NKp30 are associated with FcR γ and/or TCR ζ, while NKp44 is associated with the signaling adaptor DAP12 (EMBO J. 2004; 23:255-9).

Hemophagocytic lymphohistiocytosis (HLH) is a disease resulting from the proliferation of monocytes or macrophages and exhibiting uncontrolled hemophagocytosis and hypersecretion of inflammatory cytokines. In particular, immunodeficiency X-linked lymphoproliferative disease 1 (XLP1), which is a disease with symptoms such as HLH, occurs by infection with the Epstein-Barr virus (EBV), or in the case of congenital XLP1, a mutation of *SH2D1A,* which produces the SAP protein, forms the genetic basis of XLP1 (Annu. Rev. Immunol. 2007; 25:337-79, Annu. Rev. Immunol. 2011; 29:665-705).

2B4 contains the ITSM motif in its cytoplasmic tail and transmits an activation signal by recruiting the small adapter SAP and the SAP-associated tyrosine kinase Fyn (J. Exp. Med. 202, 181-192 (2005), Immunity 36, 974-985 (2012)). 2B4 signaling leads to the activation of Vav1, p38 MAPK, Erk, and PLC-g2 (Nat. Immunol. 9, 495-502 (2008)). Notably, in NK cells from patients with congenital XLP1, who lack functional SAP expression, 2B4 is not active, but is able to transmit an inhibitory signal (Annu. Rev. Immunol. 25, 337-379 (2007)). These NK cell defects contribute to the clinical signs of XLP1, such as Epstein-Barr virus (EBV)-infected B cell killing defects, IFN-g production defects, fulminant mononucleosis, and B-cell lymphoma.

Meanwhile, in NK cells of cancer patients, defects in NK cell activation through anticancer receptors such as NKG2D (J. Immunol. 175, 5541-5550 (2005), Cancer Res. 69, 7775-7783 (2009), J. Immunol. 189, 1360-1371 (2012)), and DNAM-1 (Immunol. Cell Biol. 90, 109-115 (2012)) are observed. It is also known that the expression and function of NKG2D and 2B4 are decreased in NK cells of patients with a hepatitis B virus infection (PloS Pathog. 8, e1002594 (2012)). Non-patent document "Minimal requirement for induction of natural cytotoxicity and intersection of activation signals by inhibitory receptors", Yenan T Bryceson ET AL BLOOD, vol. 114, no. 13, 24 September 2009 (2009-09-24), pages 2657-2666, discloses the use of recombinant insect cells expressing ligands for one or more NK cell receptors including NKG2 and 2B4 in order to asses NK activity.

Thus, NK cell activity testing is required to diagnose cancer and viral infections, in addition to HLH. However, when existing techniques using radioisotopes or fluorescently labeled probes are used to measure NK cell activity, they are unsuitable for clinical application in terms of cost and time, and thus a method capable of simply, rapidly, and significantly measuring NK cell activity is considered important for early diagnosis and prognosis determination of various immune-related diseases.

### DESCRIPTION OF EMBODIMENTS

### TECHNICAL PROBLEM

Provided is a method of testing NK cell activity, for diagnosing hemophagocytic lymphohistiocytosis or cancer including: specifically stimulating at least two distinguishable factors on NK cells in a sample; measuring a synergistic activation level of the NK cells, induced by stimulation of the at least two distinguishable factors; and comparing the synergistic activation level of the NK cells with that of normal NK cells; and wherein the NK factors used are NKG2D and 2B4 (CD244).

According to an aspect of the present disclosure, there is provided a method as claimed in claim 1.

Provided is the use of a kit for diagnosing hemophagocytic lymphohistiocytosis or cancer including: at least one selected from an antibody or fragment thereof specific to each or all of the two distinguishable factors, NKG2G and 2B4 (CD244), a target cell or a polypeptide that induces specific activity, a composition that specifically induces activity, and a reversible or irreversible agonist, as a stimulating material that stimulates at least two distinguishable factors on NK cells in a sample to thereby induce the synergistic activity of the NK cells; and a material for detecting the presence or absence of activation of the NK cells compared to normal NK cells. According to an aspect of the present disclosure, there is provided a use of a kit as claimed in claim 8.

### SOLUTION TO PROBLEM

According to an aspect of the present disclosure, , there is provided a method for diagnosing hemophagocytic lymphohistiocytosis or cancer including: specifically stimulating at least two distinguishable factors on NK cells in a sample; measuring a synergistic activation level of the NK cells, induced by stimulation of the at least two distinguishable factors and comparing the synergistic. activation level of the NK cells with that of normal NK cells. The two distinguishable NK factors are NKG2D and 2B4(CD244).

The sample mav be a biological sample derived from an individual, for example, a mammal including a human. In addition, the biological sample may be isolated from an individual and may be blood, whole blood, serum, plasma, lymph fluid, urine, feces, tissue, cells, organs, bone marrow, saliva, sputum, cerebrospinal fluid, or a combination thereof. In addition, the biological sample may include PBMCs, purified NK cells, or primary resting cells (i.e., directly isolated from blood). In one embodiment, the sample is blood.

The term "distinguishable" as used herein includes all cases where position, structure, function, and the like in a cell are not equivalent. In particular, this may indicate the necessity to differentiate signal transmission processes induced in the cell after stimulation or differentiate stimulating means or methods due to structural difference in position on the cell or between the factors themselves, or a case in which upon single stimulation, functions in the cell are different or it is insufficient to effectively and efficiently induce activity.

The term "factors" as used herein refers to all molecular elements that include the synergistic activation of NK cells. In particular, the factors may include intracellular and extracellular receptors, channels, and intracellular and extracellular adaptors, proteins, glycoproteins, peptides, motifs that are capable of binding to specific ligands.

factors may be receptors. The term "receptors" as used herein refer to all molecules that bind to a specific material to thereby change the activity of NK cells. The specific material includes a chemical composition, an *in vivo*-derived or artificial specific protein, a peptide, cholesterol, and glycoprotein, and may include cytokines or chemokines by other immune cells or NK cells themselves, or specific receptors or membrane proteins of target cells or NK cells themselves.

The factors may be present on cell surfaces or in cells. Thus, for example, when the factors are receptors, the factors include all receptors that not only induce NK cell activity by binding to a specific material on surfaces of NK cells and performing signal transduction into the cells, but are also capable of inducing signal transduction by binding to an external specific stimulating material, as receptors present in a cell membrane or cytoplasm through the cell membrane.

The term "stimulating" as used herein refers to acting on factors to cause specific signal transduction inside and outside of NK cells. The stimulating method may include using an antibody or fragment thereof, or a peptide that is specific to at least one of the factors, a specifically inducing or inhibiting composition, and a reversible or irreversible agonist or antagonist, and culturing with a culture including various target cells capable of stimulating NK cells (e.g., K562, 721.221, or P815 coated with a specific receptor stimulating antibody).

The term "synergistic" as used herein means that, although an effect of each factor is insufficient to effectively and efficiently activate NK cells, when the distinguishable factors are co-stimulated simultaneously or sequentially, significant changes may be caused or NK cell activity may be effectively and efficiently induced, as compared to a case in which the factors are each independently stimulated. Thus, the synergistic activity or synergistic activation of NK cells may be determined such that each of the at least two distinguishable factors on the NK cells and a combination thereof are specifically stimulated, the activity of the NK cells, which is induced by stimulation of each factor and the combination thereof, is measured, and a case in which an NK cell activation level by stimulation of the combination thereof is at least 1.5 times, at least 2 times, or at least 3 times, preferably 2 times, that by stimulation of each factor (preferably, a higher NK cell activation level among those of the factors) is determined as induction of the synergistic activation. As described in the present specification, the synergistic activation level may be quantified by flow cytometry analysis, immunoblotting, or the like, based on the measurement of a phosphorylation or expression level of a sub-factor, degranulation activity, cytotoxic activity, and cytokines secreted by NK cell stimulation, but the present disclosure is not limited thereto.

In the method for diagnosing hemophagocytic lymphohistiocytosis or cancer of the present disclosure, the comparing of the synergistic activation level of the NK cells with that of normal NK cells includes, in particular, determining that, when each of at least two distinguishable factors or a specific combination thereof of normal NK cells as a control are stimulated by under the same condition as that of an experimental group, a case in which a synergistic activation level shown in the normal NK cells is significantly lower or higher than that of the experimental group, or synergistic activation phenomena are not shown in the experimental group is determined as abnormal. In addition, in the case of co-stimulation, comparison is easily performed as compared to the case of stimulation of a single factor, and thus the comparing process includes performing determination by artificially inducing synergistic activation and comparing data with each other. Through this process, pathological indications of abnormal NK cell-associated diseases, viral infections, the presence of cancer cells, and specific cancer may be determined and prognosis for these diseases may be predicted. The term "normal NK cells" as used herein refers to NK cells of a disease-free individual or NK cells derived from such an individual, and the disease-free individual has at least no physical, genetic or exotic conditions known to affect NK cell activity.

The method may further include isolating target NK cells from the sample. If needed, this process may be performed after stimulation in the presence of NK cells together with other hemocytes or lymphocytes, or this process may be performed before the stimulating process in the case of a sample containing only NK cells as lymphocytes. A purification level of the isolated NK cells and the composition of the sample may vary according to the degree of necessity in experiments. NK cells may be, if needed, directly used in a form purified in a sample or used after proliferation to secure conditions or cell amount suitable for an experiment.

However, a combination of specific factors is specific to NK cells (see Example 6), and thus when the combination of NK cell-specific factors is used, the isolating of the NK cells may not be necessarily performed.

The stimulating process may be performed on the factors simultaneously or sequentially. In particular, the stimulating process may be performed by structural binding to the factors or modification of the factors. The modification may include, for example, post-translation modification or cleavage, such as phosphorylation, ubiquitination, sulfonylation, methylation, parylation, or glycosylation.

The are selected from NKG2D, 2B4 (CD244). According to Bryceson Y et al. (Blood 2006;107:159-166) and Kim HS et al. (Immunity 2010;32:175-186), NK cell synergistic activation may be enabled by various combinations of factors, and examples of the combination of the factors include 2B4 and DNAM-1; DNAM-1 and NKG2D; 2B4 and NKG2D; and 2B4, DNAM-1, and NKG2D.

In the stimulating process, the stimulation may be induced by at least one selected from an antibody or fragment thereof specific to each or all of the at least two factors, a target cell or a polypeptide that induces specific activity, a specifically inducing or inhibiting compound, and a reversible or irreversible agonist or antagonist. The factor-specific antibody or fragment thereof includes isoforms and may simultaneously bind to the at least two distinguishable factors. In particular, examples of the factor-specific antibody or fragment thereof simultaneously binding to the at least two distinguishable factors may include P815 coated with CD244/2B4 or CD226/DNAM-1. The agonist or antagonist may be a compound with structural specificity to a target factor and may be a polypeptide having tertiary structural specificity.

Target cell that induces specific activity with respect to the factors may be coated with at least one selected from an antibody against at least two distinguishable factors on NK cells, a fragment, and a combination thereof. The at least two distinguishable factors on NK cells are the same as those described above. The antibody may be an antibody against isoforms of the factors, and the fragment may be a fragment of the antibody and include only a specific region of the antibody sufficient to induce activity. The coating process may be performed by preincubation with target cells as described in the following examples, or may include coating with the antibody, the fragment, or a combination thereof by cross-linking.

Measuring of the synergistic activation level may include measuring at least one selected from a phosphorylation or expression level of a sub-factor of each of the at least two distinguishable factors on NK cells, degranulation activity, cytotoxic activity, and cytokines secreted by NK cell stimulation. The term "sub-factor" as used herein refers to an intracellular protein or a changed polypeptide, the expression of which is increased or which is phosphorylated when the factors on NK cells are stimulated, and in particular, in the case of phosphorylation, the sub-factor may be used in the same sense as a substrate.

An expression level of the sub-factor may be measured by, for example, immunoblotting, which is well known in the art, using an antibody specific to Erk or pY174-Vav1, which is one of the intracellular proteins involved in NK cell activation, after the stimulated NK cells are lysed.

The degranulation activity may indicate, for example, induction of the lysis of target cells by secretion of perforin or granzyme and may be analyzed by FACS. In particular, a method of measuring CD107a expression, which is proportional to degranulation, using an fluorochrome-conjugated antibody, after PBMCs isolated from a sample or purely isolated NK cells are stimulated may be used.

The cytotoxic activity may be measured by, for example, culturing NK cells labeled with a europium fluorescent dye together with a culture containing target cells capable of activating the NK cells, and then measuring the amount of the fluorescent dye released through target cell lysis by using a microplate reader.

In one embodiment, the cytokines may be selected from IFN-_{Y}, TNF-α, TNF-β, MIP-1α, MIP-1β, PANTES, IL-8, and IL-10. The expression analysis of the immunostimulatory factors of NK cells may be performed by FACS, intracellular cytokine staining, ELISA, or the like. In particular, a method of measuring the expression of an immune activating factor in NK cells by staining surfaces of the NK cells with fluorochrome-conjugated specific antibodies, permeabilizing the cells, and then staining the cytokines or the like with fluorochrome-conjugated other specific antibodies against an immune activating factor (e.g., IFN-_{Y}) may be used.

When synergistic activation was induced in normal people based on a higher one of the levels of the two factors, the normal people showed an at least two-fold difference in the secretion amounts of cytokines or a cytotoxic degranulation level, as compared to patients, and even in the case of comparison after the synergistic activation of NK cells was induced, the normal people showed an at least two-fold difference in the secretion amounts of synergistically activated cytokines or a cytotoxic degranulation level.

Thus, in the comparing of the synergistic activation level of the NK cells compared to that of normal NK cells, a case in which differences in the phosphorylation or expression level of the sub-factor, degranulation activity, cytotoxic activity, and the secretion amounts of cytokines secreted by NK cell stimulation are at least two times, particularly at least two times a higher one of levels of two factors may be determined that NK cell activity in the sample is normal. In other embodiments, a case in which a difference in the secretion amounts of synergistically activated cytokines of the NK cells or a cytotoxic degranulation level of the NK cells is two times or less, particularly, two times or less the secretion amounts of synergistically activated cytokines of the normal NK cells or a cytotoxic degranulation level of the normal NK cells may be determined that NK cell activity in the sample is abnormal.

The inventors of the present disclosure verified that, although the activation of NK cells was induced in patients with HLH and pancreatic cancer, normal people showed a high level of synergistic activation, whereas the patients failed to show synergistic activity or showed a significantly low degree thereof. In particular, they found that, in the case of comparison after synergistic activation was induced, the diagnosis of HLH or pancreatic cancer showed an increased significance and was more easy and accurate, as compared to the case of comparison after NK cell activation was simply induced. Thus, the method of testing NK cell activity of the present disclosure distinguishes a sample showing abnormal NK cell synergistic activity, and thus may be usefully used in providing information on the diagnosis of NK cell synergistic activity-associated diseases including HLH and pancreatic cancer.

The NK cell synergistic activity-associated diseases are histiocytosis and cancer. The hystiocystosis is HLH. Hemophagocytic lymphohistiocytosis (HLH) may include the meaning of Class II Langerhans cell histiocytosis, erythrophagocytic lymphohistiocytosis (familial and sporadic), infection-associated hemophagocytic syndrome, virus-associated hemophagocytic syndrome, histiocytosis accompanied by massive lymphadenopathy, or reticulohistiocytosis. The term "cancer" as used herein is intended to include tumors, blood cancer, or solid cancer, and includes a condition where the NK cell synergistic activity of an individual is impaired or the synergistic activity of NK cells against target cells is not induced under specific conditions. In one embodiment, the cancer may be selected from the group consisting of lung cancer, liver cancer, esophageal cancer, stomach cancer, colon cancer, small intestine cancer, pancreatic cancer, melanoma, breast cancer, oral cancer, brain tumor, thyroid cancer, parathyroid cancer, kidney cancer, cervical cancer, sarcoma, prostate cancer, urethral cancer, bladder cancer, testicular cancer, blood cancer, lymphoma, skin cancer, psoriasis,and fibroadenoma. In one embodiment, the cancer may be pancreatic cancer or B cell lymphoma. In one embodiment, the virus disease may be hepatitis B. In embodiment, the immunodeficiency disease may be DiGeorge syndrome or Chedial-Higashi syndrome.

According to the information on NK cell synergistic activity-associated diseases, a case in which the synergistic activity of NK cells as an experimental group compared to normal NK cells is not detected may be determined as abnormal, or a case in which NK cells having lost synergistic activity for a specific receptor abnormally exhibit or do not exhibit synergistic activity with respect to target cells may be determined that the target cells are associated with a specific disease. To further facilitate the diagnosis of the specific disease, the method may further include coating the target cell with at least one selected from an antibody against the at least two distinguishable factors on NK cells, a fragment, and a combination thereof.

According to another aspect of the present disclosure, there is provided the use of a kit for diagnosing hemophagocytic lymphohistiocytosis or cancer including: at least one selected from an antibody or fragment thereof specific to each or all of at least two distinguishable factors, NKG2D and 2B4(CD244), a target cell or polypeptide that induces specific activity, a composition that specifically induces activity, and a reversible or irreversible agonist, as a stimulating material that stimulates at least two distinguishable factors on NK cells in a sample to thereby induce the synergistic activity of the NK cells; and a material for detecting the presence or absence of activation of the NK cells compared to normal NK cells.

The material for detecting the presence or absence of synergistic activation of the NK cells may be at least one selected from: an antibody against at least one selected from perforin, granzyme, CD107a, CD107b, IFN-γ, MIP-1α, MIP-1β, TNF-α, TNF-β, PANTES, IL-8, and IL-10; a fragment; and a combination thereof, or a fluorescent dye. The antibody, the fragment, or a combination thereof may be labeled or not labeled with a fluorescent probe. The fluorescent dye or the fluorescent probe may vary according to conditions such as a subject to be detected, the combination or number of the subjects, a desired wavelength, a detector, and the like, and may be, for example, peridinin chlorophyll (PerCP), phycoerythrin (PE), fluorescein isothiocyanate (FITC), or europium, but the present disclosure is not limited thereto. According to one embodiment of the present disclosure, IFN-_{Y} and TNF-α may be directly detected using a flow cytometer by being labeled with FITC, which is a fluorescent probe, without secondary treatment. In addition, the activation of NK cells against target cells (e.g., P815) stained with a fluorescent dye, the fluorescent dye released by lysis of the target cells may be measured, thereby measuring a synergistic activation level of the NK cells. Thus, the use of a fluorescent dye as the material for detecting the presence or absence of synergistic activation of the NK cells may be suitable particularly for cytotoxicity measurement.

In one embodiment, the target cell that induces activity specific to the factors on NK cells may be coated with at least one selected from an antibody against the at least two distinguishable factors on NK cells, a fragment, and a combination thereof.

In one embodiment, the use of the kit for diagnosis of hemophagocytic lymphohistiocytosis or cancer may further include, as a substance for visually displaying detection results showing the presence or absence of synergistic activation of the NK cells, a dye, a fluorescent dye, a secondary antibody, or a biological or molecular probe.

The NK cell synergistic activity-associated disease is histiocytosis or cancer.

It is to be understood that the terms or elements mentioned above with respect to the kit are the same as those mentioned in the description of the claimed method for diagnosing hemophagocytic lymphohistiocytosis or cancer.

### ADVANTAGEOUS EFFECTS OF DISCLOSURE

By using a method for diagnosing hemophagocytic lymphohistiocytosis or cancer according to an embodiment, including: specifically stimulating the distinguishable factors NKG2D and 2B4(CD244) on NK cells in a sample; measuring a synergistic activation level of the NK cells; and comparing the synergistic activation level of the NK cells with that of normal NK cells, the NK cell activity may be tested more simply and rapidly than previously known techniques, and significantly, and thus early diagnosis and prognosis determination of NK cell activity-associated diseases may be easily performed.

By using a kit for diagnosing hemophagocytic lymphohistiocytosis or cancer according to another embodiment, including: at least one selected from an antibody or fragment thereof specific to each or all the two distinguishable factors NKG2D and 2B4(CD244) on NK cells, a target cell or a polypeptide that induces specific activity, a composition that specifically induces activity, and a reversible or irreversible agonist, as a stimulating material that stimulates at least two distinguishable factors on NK cells in a sample to thereby induce the synergistic activity of the NK cells; and a material for detecting the presence or absence of activation of the NK cells compared to normal NK cells, the early diagnosis or prognosis determination of NK cell activity-associated diseases may be performed more simply and rapidly than previously known techniques and significantly.

### BRIEF DESCRIPTION OF DRAWINGS

FIG. 1 illustrates flow cytometry analysis results showing a phosphorylation level of the NF-_{K}B p65 subunit upon specific stimulation of a distinguishable factor, i.e., NKG2D and/or 2B4.
FIG. 2 illustrates flow cytometry analysis results showing the frequency of cells expressing IFN-_{Y} or TNF-α upon specific stimulation of a distinguishable factor, i.e., NKG2D and/or 2B4.
FIG. 3 illustrates results of identifying the synergistic activity of NK cells through cytotoxicity when a specific receptor is stimulated alone or specific receptors are co-stimulated.
FIG. 4 illustrates expression levels of NK cell activation-associated factors when NK cells obtained from normal people or XLP1 patient donors were activated by stimulation of a distinguishable factor, i.e., NKG2D and/or 2B4.
FIG. 5A illustrates flow cytometry analysis results showing an expression level of IFN-_{Y} when NK cells obtained by normal people or XLP1 patient donors were activated by stimulation of the distinguishable factor NKG2D and/or 2B4; and FIG. 5B illustrates the percentage of an increase in NK cells expressing IFN-γ upon stimulation (IFN-_{Y}+ NK cells) as compared to when NK cells were not stimulated (ΔIFN-γ+ cells).
FIG. 6 illustrates defective cytotoxic degranulation of XLP1 NK cells against co-activation, wherein FIG. 6A illustrates flow cytometry analysis results showing an expression level of CD107a when NK cells obtained from normal people or XLP1 donors are activated by stimulation of the distinguishable factor NKG2D and/or 2B4; and FIG. 6B illustrates the percentage of an increase in CD107a+ NK cells upon stimulation (CD107a+ NK cells) as compared to when NK cells were not stimulated (ΔCD107a+ cells).
FIG. 7 illustrates flow cytometry analysis results showing activation levels in various hemocytes by stimulation of a combination of NKG2D/2B4.
FIG. 8 illustrates flow cytometry analysis results showing activation levels in NK cells by stimulation of a combination of NKG2D/2B4.

### MODE OF DISCLOSURE

Hereinafter, the present disclosure will be described in further detail with reference to the following examples.

### Example 1. Identification of Presence or Absence of Synergistic Activation of Normal NK Cells by Co-activation of Distinguishable Factors NKG2D and 2B4

### 1-1. NK Cell Acquisition

Human blood samples were collected for research purposes from healthy donors and XLP1 donors in accordance with notification under protocols approved by the Asan Medical Center, the UCL institute of Child Health, and the Institutional Review Board of the Philadelphia Pediatric Hospital. Peripheral blood mononuclear cells (PBMCs) were isolated from the blood samples using a density-gradient centrifuge (lymphocyte separation medium (LSM)) (using MP Biomedicals), and then cryopreserved during this process. Human NK cells were purified from the PBMCs by negative selection using an NK cell isolation kit (StemCell Technologies) as previously known (Sci. Signal. 5, ra49 (2012)). These cells were 97% to 99% CD3-CD56+ as evaluated by flow cytometry. The human NK cell line NKL (donated by M.Robertson) was cultured in RPMI1640 supplemented with 10% FBS, 1 mM sodium pyruvate, and 200 U/ml recombinant IL-2 (rIL-2). NKL cells were rested in RPMI1640 supplemented with 5% FBS and 0.5 mM sodium pyruvate for 24 hours without rIL-2.

### 1-2. Phosphorylation of NF-_{K}B p65 Subunit as Indicator of Synergistic Activity of NK Cells by Co-activation of NKG2D and 2B4

NK-_{K}B is an important transcription factor as a regulator in congenital and acquired immune responses, and when the Ser536 residue of p65, which is the NK-_{K}B subunit, is phosphorylated, the activity of NK cells is increased (Front Immunol. 2014; 5: 662.). Thus, phosphorylation of the NF-_{K}B p65 subunit in primary resting NK cells was measured as an NK cell activation factor.

The following receptor crosslinking was performed to stimulate specific factors inducing the activation of NK cells.

Purified human primary NK or NKL cells were preincubated with mAb specific to NKG2D and/or 2B4, which are NK receptors (10 µg/ml for all), or isoform mAb as a control (clgG1) on ice for 30 minutes. After washing with a medium, the NK cells were stimulated by receptor crosslinking with goat anti-mouse F(ab')2 secondary Ab at 37 °C for 5 minutes. The cells were washed with DPBS and fixed in 4 % paraformaldehyde at 37 °C for 10 minutes. Thereafter, the fixed cells were permeabilized with 90 % methanol on ice for 30 minutes and blocked with 0.5 % BSA at room temperature for 10 minutes. The cells were stained with Alexa Fluor 488-conjugated anti-pS536 p65 Ab (93H1) or isoform control rabbit IgG (DA1E) (Cell Signaling), and then p65 phosphorylation was analyzed using a flow cytometry analyzer.

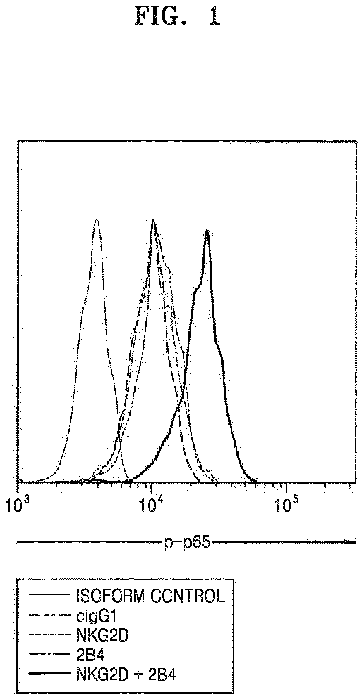

As a result, as illustrated in FIG. 1, the proportion of responding cells was synergistically increased by co-stimulation of NKG2D and 2B4 of primary NK cells, while being significantly decreased by stimulation of a receptor alone compared to co-activation of the receptors.

### 1-3. Measurement of Immune Activating Factor as Indicator of Synergistic Activity of NK Cells by Co-activation of NKG2D and 2B4

Upon activation, NK cells secrete IFN-γ, TNF-α, or the like, which is an immune activating factor (or cytokines). Thus, it was tested whether the secretion amounts of cytokines were synergistically increased by co-activation of distinguishable specific factors.

As described above, NK cells were collected, and PBMCs were stimulated with P815 cells (American Type Culture Collection) having been preincubated with mAb specific to NKG2D and/or 2B4 or isoform control mAb. Thereafter, incubation was performed for 6 hours, and cell surface markers were stained with CD3 and CD56. To identify the production of cytokines by NK cells, the expression of IFN-_{Y} or TNF-α in CD3-CD56+ NK cells was measured using a flow cytometer. In particular, PBMCs or primary resting NK cells were stimulated with the same number of predetermined target cells at 37 °C for 1 hour. Thereafter, brefeldin A (GolgiPlug; BD Bioscience) was added thereto and an additional 5 hour-long incubation was performed for a total of 6 hours. Subsequently, the surface markers were stained with anti-CD56-PE and/or anti-CD3-PerCP mAbs in a dark room at 4 °C for 30 minutes. After the cells were washed twice with FACS buffer, the washed cells were incubated in a BD Cytofix/Cytoperm solution (BD Bioscience) in a dark room at 4 °C for 20 minutes. Before and after the cells were subjected to intracellular staining with anti-IFN-y-FITC or anti-TNF-a-FITC mAb in a dark room at 4 °C for 30 minutes, the cells were washed twice with BD Perm/Wash buffer (BD Bioscience). The cells were then gated for NK cells and analyzed using a flow cytometer.

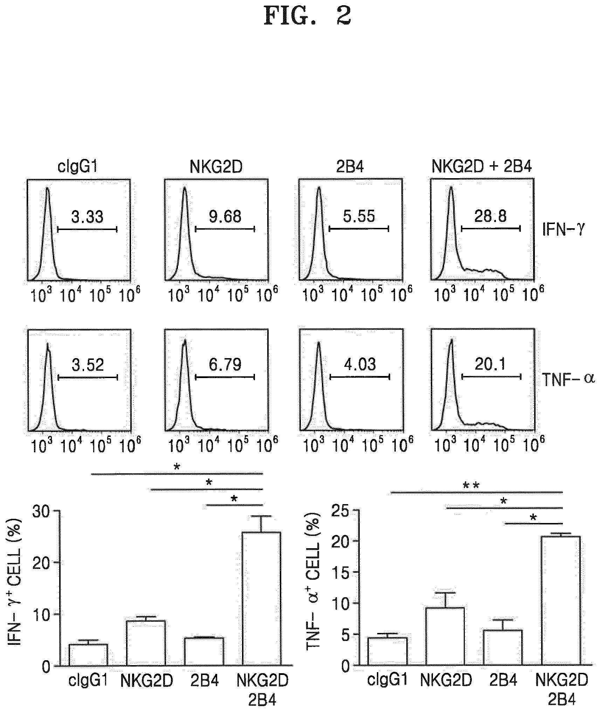

As a result, as illustrated in FIG. 2, it was confirmed that the co-engagement of NKG2D and 2B4 induced a synergistic increase in the proportion of NK cells expressing IFN-_{Y} or TNF-α, which is an immune activating factor.

Taken altogether, these results indicate that it is not sufficient to stimulate NF-_{K}B activation to be effective on NK cell activity by single stimulation of each of NKG2D and 2B4, and the threshold for NF-_{K}B activation is overcome by co-activation of NKG2D and 2B4, thus inducing the synergistic production of cytokines by NK cells.

Each graph was constructed by at least three independent experiments. The results were expressed as mean ± SD. Statistical analysis was performed by a two-tailed student's *t*-test using the GraphPad Prism software.

### Example 2. Measurement of Cytotoxicity as Indicator of Synergistic Activity of NK Cells by Co-activation of Distinguishable Factors

As it was observed that a synergistic increase in the proportion of NK cells expressing IFN-_{Y} or TNF-α, which is an immune activating factor, was induced by the co-activation of NKG2D and 2B4, it was also tested whether a synergistic increase in cytotoxicity, which is a method of measuring synergistic activity, was induced.

P815 cells were preincubated with mAb specific to CD56, CD16 and NKG2D, and/or 2B4, DNAM-1, and/or 2B4; or isoform control mAb, and the P815 cells, which were coated with the NK cell specific receptor stimulating antibody, was labeled with a europium fluorescent dye. Subsequently, as described above, primary resting NK cells were stimulated by incubation together with the P815 cells. Thereafter, a culture was recovered by centrifugation and the amount of the fluorescent dye released through target cell lysis, which is an indicator of cytotoxicity, was measured. The degree of fluorescence in the culture was analyzed using a microplate reader.

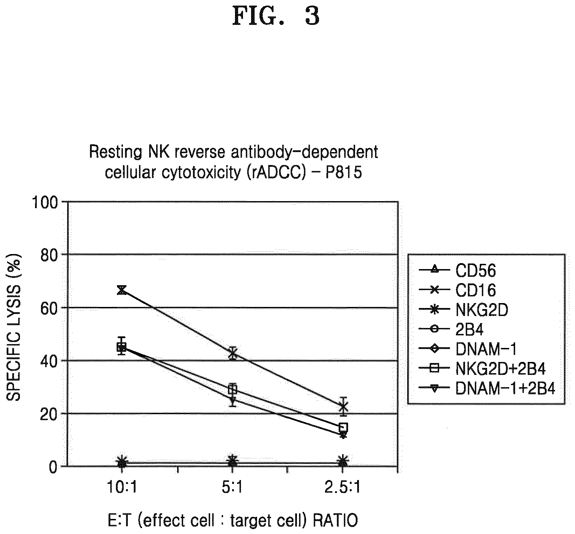

As a result, as illustrated in FIG. 3, it was confirmed that although CD16 exceptionally induced strong cytotoxicity simply, cytotoxicity was synergistically induced by co-stimulation of NKG2D+2B4 and DNAM-1+2B4, compared to the case of stimulation of each factor. Although there may be exceptional cases, these cases correspond to specific receptor stimulation (e.g., using the aforementioned specific receptor stimulating antibody) and thus may be excluded. Thus, it was confirmed that not only synergistic activity measurement could be realized by specifically stimulating two distinguishable factors, but synergistic activity induced by co-stimulation also had a significant difference in comparison for NK activity, as compared to single stimulation, thus being easily distinguishable.

### Example 3. NK Cell Activity Comparison Using Synergistic Difference in Specific Factor Expression by Co-activation of NKG2D and 2B4 of Normal People and XLP1 Patients

It was examined whether the synergistic activity of NK cells could be induced by specifically co-activating NKG2D and 2B4, which are distinguishable factors. Thus, to confirm whether patients with XLP1, which is a HLH disease, could be diagnosed using the presence or absence of the synergistic activity, western blotting from which a synergistic difference between expression levels of specific factors can be confirmed was performed.

### 3-1. NK Cell Acquisition of XLP1 Patients

As described above, NK cells were donated from patients with XLP1. Here, patients with hereditary *SH2D1A* gene mutation were included as a subject of congenital XLP1. Due to limitations in supplying patient NK cells, NK cells of XLP1 patients were proliferated as described above to study molecular signaling for NF-_{K}B activation and effector functions. NK cells of normal donors were also proliferated, and stimulated with NKG2D and 2B4 after the resting period to reproduce synergistic increases in effector functions and signaling.

Western blotting was performed to determine whether NK cells of XLP1 patients could be an indicator of diseases. According to a previous report (Nat. Rev. Immunol. 6, 56-66 (2006)), SAP is a receptor adaptor protein required to transmit activation signals by immunostimulation, including 2B4, and it is known that an expression amount of SAP in NK cells is depleted or decreased in XLP1 patients. A whole lysate of primary NK cells proliferated from normal people or XLP1 patient donors was western blotted to SAP and actin, and expression amounts thereof were examined, from which it was confirmed that, among four tested XLPL patients, three patients had macrodeletion in the *SH2D1A* gene inducing the complete loss of SAP expression, and one patient had missense mutation where SAP expression is decreased, but is not completely lost.

### 3-2. Comparison between Synergistic Expression Levels of ErK and Phosphorylated p65

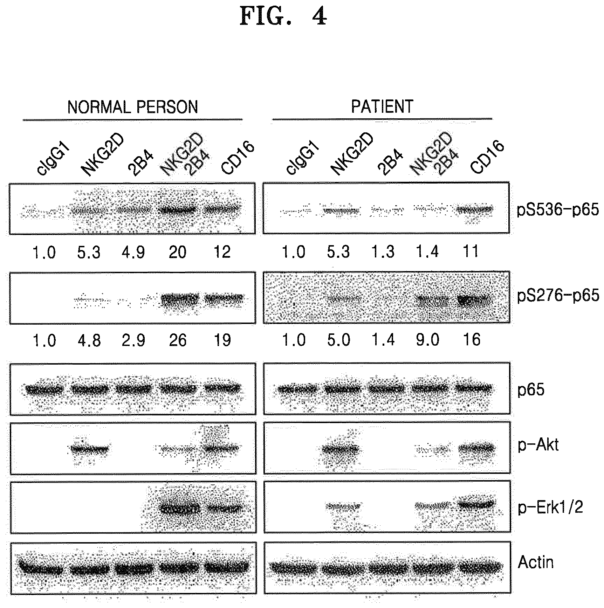

A difference in activity between normal NK cells and NK cells of XLP1 patients was compared by western blotting. In particular, primary resting NK cells, which were obtained from normal people or XLP1 patient donors and proliferated, were preincubated with mAb specific to predetermined receptors or isoform control mAb (clgG1). Receptor crosslinkding was performed for 2 minutes (p-Akt and p-Erk1/2) and 5 minutes (pS536-p65 and pS276-p65), and then the resulting lysate was immunoblotted by predetermined phosphorylation. As a result, as illustrated in FIG. 4, although the phosphorylation of p65 by NF-_{K}B activation, which is an indicator of NK cell activity, is observed by stimulation of each of NKG2D and 2B4, the phosphorylation level is not significantly distinguished from synergistic phosphorylation by the co-activation of NKG2D and 2B4. In contrast, the synergistic phosphorylation of the Ser536 and Ser276 of p65 may be confirmed to be significantly decreased in the XLP1 NK cells by the co-activation of NKG2D and 2B4. This was also shown in the synergistic phosphorylation of Erk induced by co-activation, from which it was confirmed that the synergistic phosphorylation was significantly decreased in the XLP1 NK cells as compared to the normal NK cells. Thus, in diagnosis of XLP1, when NK cell activity is measured by co-activation compared to stimulation of each of distinguishable factors, a significant difference in synergistic phosphorylation is observed, and this suggests that XLP1 may be effectively diagnosed based on the synergistic activity induced by co-activation of distinguishable factors of an NK cell.

### Example 4. NK Cell Activity Comparison Using Synergistic Difference in Expression Amount of IFN-γ by Co-activation of NKG2D and 2B4 of Normal People and XLP1 Patients

To investigate whether comparison in synergistic activity due to stimulation of two distinguishable factors is more suitable for XLP1 diagnosis, an experiment for comparing expression amounts of IFN-_{Y}, which is an immune activating factor, was carried out. In particular, primary resting NK cells, which were obtained from normal people or XLP1 patient donors and proliferated, were mixed with P815 cells preincubated with mAb specific to predetermined receptors. After 6 hour-long incubation, the resulting mixture was subjected to staining with fluorochrome-conjugated mAb against CD56 and intercellular staining with IFN-γ, followed by flow cytometry analysis as described above.

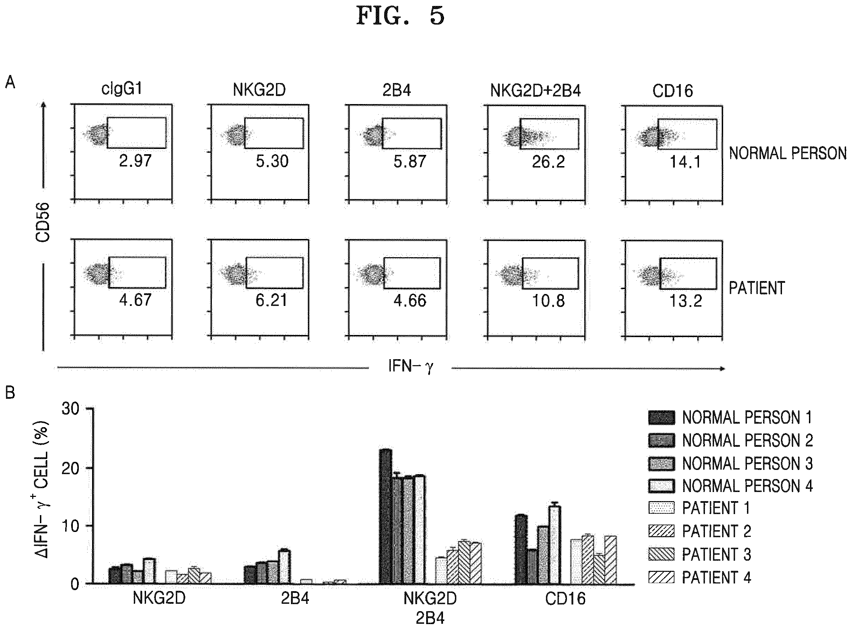

As a result, as illustrated in FIG. 5, while CD16 stimulation had no significance in NK cell activation in XLP1, the proportion of NK cells expressing IFN-_{Y} was significantly decreased in the XLP1 NK cells by co-stimulation of NKG2D and 2B4. As illustrated in FIG. 5B, the difference was measured as the percentage of an increase in IFN-_{Y}+ NK cells from each of normal people or XLP1 patient donors after being stimulated with a predetermined receptor with respect to non-stimulated cells (ΔIFN-γ+ cells). It can be seen that synergistic activity shows a more significant and distinct difference by co-stimulation of NKG2D and 2B4, as compared to single stimulation of each of NKG2D and 2B4. This also suggests that when comparison is performed after synergistic activation is induced, more significant determination may be made, as compared to when comparison is performed after single stimulation.

In summary, these results demonstrate that genetic and molecular differences in NK cells of normal people and XLP1 patients are consistent with the results for co-activation stimulation, and determination of the conditions of factors applied to the above experiments has ample grounds for XLP1 diagnosis. In addition, these results suggest that comparison in synergistic activity induced by stimulating at least two factors capable of inducing synergistic activation may be a method of more significantly diagnosing XLP1. Moreover, the above results showing synergistic phosphorylation shown only in a specific factor (Erk) and synergistic activation by the combination of specific receptors (NKG2D and 2B4) demonstrate that the specific co-activation of specific receptors is suitable for use in diagnosing at least XLP1 and related clinical prognosis prediction.

Each graph was constructed by at least three independent experiments. The results were expressed as mean ± SD. Statistical analysis was performed by a two-tailed student's t-test using the GraphPad Prism software.

### Example 5. NK Cell Activity Comparison Using Synergistic Difference in Degranulation by Co-activation of NKG2D and 2B4 of Normal People and XLP1 Patients

It was examined whether XLP1 could be diagnosed using a difference in synergistic effect induced by stimulation of two distinguishable factors by measuring an expression level of CD107a on NK cell surfaces, proportional to degranulation, which is an indicator of NK cell activity.

The degranulation of NK cells was evaluated by CD107a expression on cell surfaces and Granzyme B release (BioLegend). In summary, as described above, primary resting NK cells of normal people and XLP1 patients were proliferated, and then mixed with P815 preincubated with mAb specific to a predetermined receptor in the presence of fluorochrome-conjugated anti-CD107a mAb. The resulting mixture was then incubated for 2 hours and analyzed using a flow cytometer. In particular, the resulting mixture was spun down at 30 x g for 3 minutes, incubated at 37 °C for 2 hours, and spun down again. The cell pellet was re-suspended in FACS buffer (PBS containing 2% FBS) and stained with anti-CD56-PE in a dark room at 4 °C for 30 minutes. Lymphocytes were gated by forward scatter/side scatter and CD107a expression in NK cells was analyzed using a flow cytometer and the FlowJo software.

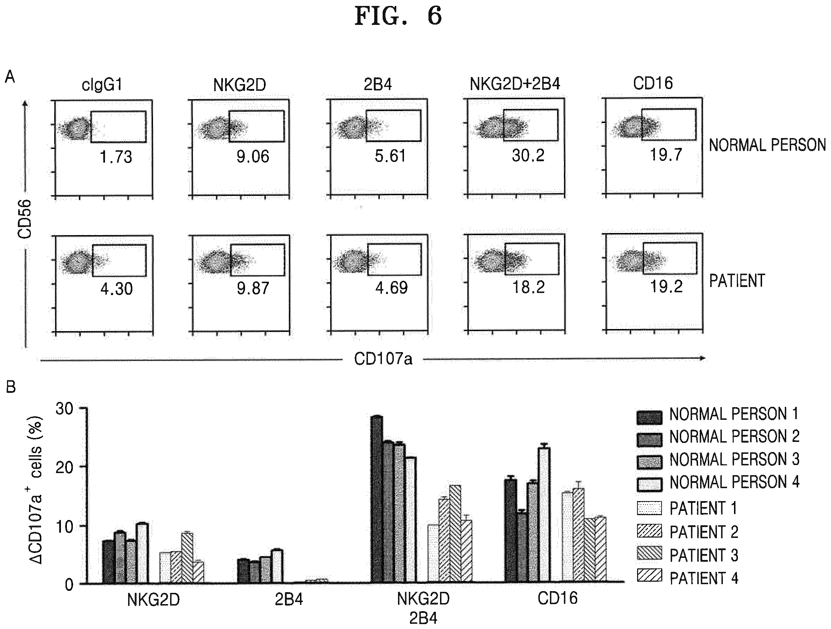

As a result, as illustrated in FIG. 6A, although no significant difference is observed in degranulation, which is an indicator of synergistic activity, upon stimulation of CD16, as compared to the normal NK cells, degranulation showing a significant difference is induced by co-stimulation of NKG2D and 2B4. From these results, it can also be seen that similarly to the case of the measurement of IFN-γ, which is an immune activating factor, it is more significant as a diagnosis method to determine the presence or absence of a significant difference after synergistic activity is induced by co-activation, rather than comparison after stimulation of each of NKG2D and 2B4. This can be more clearly seen in the group of FIG. 6B, showing the percentage of CD107a+ NK cells with respect to non-stimulated cells, ΔCD107a+ cells.

### Example 6. Evaluation of NK Cell Activity Specificity by Co-activation of NKG2D and 2B4

To determine whether synergy measurement using NKG2D/2B4 combination is specific to only NK cells among other cells, the expression level of CD107a on NK cell surfaces, proportional to degranulation, which is an indicator of NK cell activity, was measured.

In particular, normal human mononuclear cells (PBMCs) were mixed with P815 cells expressing ligands (ULBP1 for NKG2D and CD48 for 2B4) specific to predetermined receptors in the presence of fluorochrome-conjugated anti-CD107a mAb. Subsequently, as in Example 5, each cell pellet was collected and re-suspended, and stained with anti-CD3-PerCP and anti-CD56-PE in a dark room at 4 °C for 30 minutes. Lymphocytes were gated by forward scatter/side scatter, and CD107a expression in NK cells (CD3-CD56+), NKT cells (CD3+CD56+), T cells (CD3+CD56-), and the remaining cells (CD3-CD56-) was analyzed using a flow cytometer and the FlowJo software.

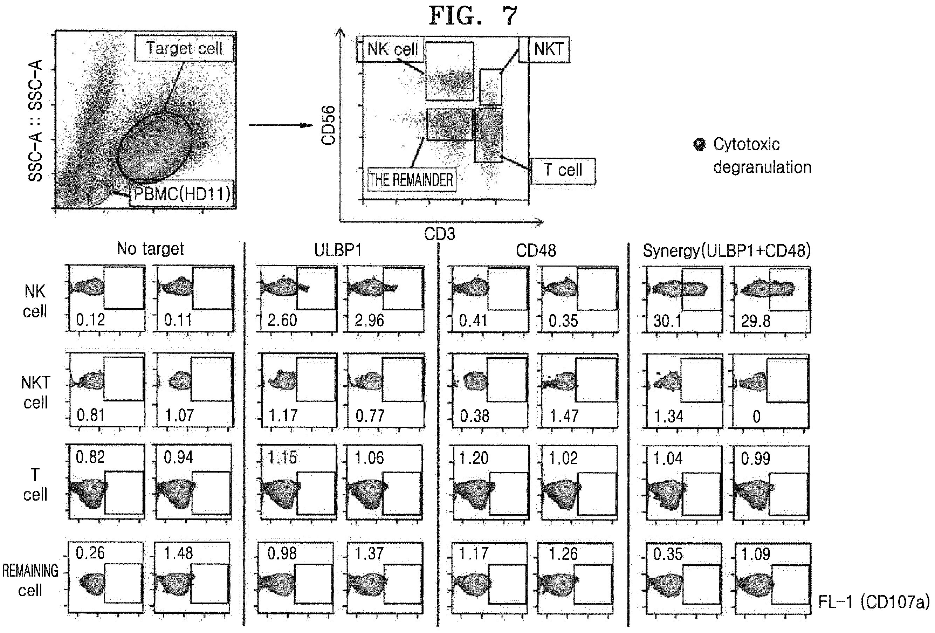

As a result, as illustrated in FIG. 7, although degranulation was not observed in all of the other cells including the NK cells upon single stimulation of each of NKG2D and 2B4, the induction of degranulation showing a NK cell-specific significant difference was observed by co-stimulation of NKG2D and 2B4. Thus, it can be seen that it is more significant as a diagnosis method to induce NK cell-specific activation by synergistic activity induced by co-activation, rather than comparison after single stimulation of each of NKG2D and 2B4.

### Example 7. NK Cell Synergistic Activity Comparison by Co-activation of NKG2D and 2B4 in Cancer Patients

To determine whether synergy measurement using NKG2D/2B4 combination is applicable to cancer diagnosis, the expression level of CD107a on NK cell surfaces, proportional to degranulation, which is an indicator of NK cell activity, was measured using pancreatic cancer patient samples.

In particular, mononuclear cells (PBMCs) of normal people and pancreatic cancer patients was mixed with P815 cells expressing ligands (ULBP1 for NKG2D and CD48 for 2B4) specific to predetermined receptors in the presence of fluorochrome-conjugated anti-CD107a mAb. As a control, the blood cancer cell line K562, which is used in measuring NK cell activity, was used. Subsequently, as in Example 5, each cell pellet was collected and re-suspended, and stained with anti-CD3-PerCP and anti-CD56-PE in a dark room at 4 °C for 30 minutes. Lymphocytes were gated by forward scatter/side scatter, and CD107a expression in NK cells (CD3-CD56+) was analyzed using a flow cytometer and the FlowJo software.

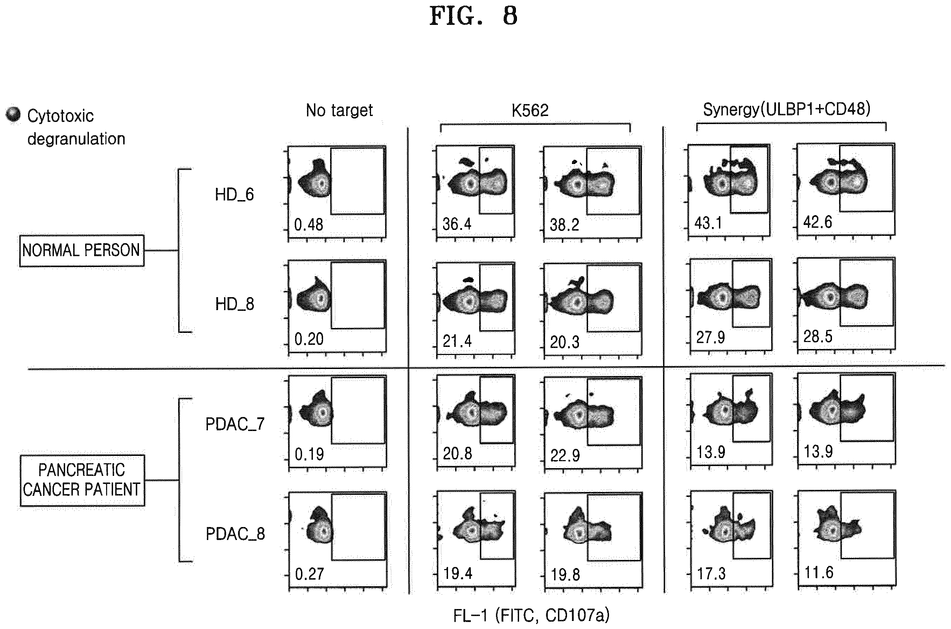

As a result, as illustrated in FIG. 8, the induction of NK cell degranulation showing a significant difference in normal people and pancreatic cancer patients can be observed by co-stimulation of NKG2D and 2B4, and thus it can be seen that synergy measurement using NKG2D/2B4 combination is also applicable to cancer diagnosis.

Specifically, no significant difference in the induction of NK cell degranulation was observed in normal people and pancreatic cancer patients when NK cells were stimulated with K562 as a control, unlike the case of specific stimulation of NKG2D and 2B4. Thus, it can be seen that synergistic activation by stimulation of specific factors on NK cells, such as co-stimulation of NKG2D and 2B4, is more significant as a method of diagnosing NK cell activity rather than using K562.

## Claims

1. A method for diagnosing hemophagocytic lymphohistiocytosis (HLH) or cancer in a sample, the method comprising:
specifically stimulating two distinguishable factors on NK cells in a sample;
measuring a synergistic activation level of the NK cells, induced by stimulation of the two factors; and
comparing the synergistic activation level of the NK cells with that of normal NK cells wherein the two distinguishable factors are NKG2D and 2B4 (CD244).

2. The method of claim 1, wherein the factors are present on surfaces of the NK cells or in the NK cells.

3. The method of claim 1, wherein the stimulating is performed on the factors simultaneously or sequentially.

4. The method of claim 1, wherein the stimulating is performed by at least one selected from an antibody or fragment thereof specific to each or all of the factors, a target cell or polypeptide that induces specific activity, a compound that specifically induces activity, and a reversible or irreversible agonist.

5. The method of claim 4, wherein the target cell that induces specific activity in the factors is coated with at least one selected from an antibody against the two distinguishable factors on NK cells, a fragment thereof, and a combination thereof.

6. The method of claim 1, wherein the measuring comprises measuring at least one selected from a phosphorylation or expression level of a sub-factor of each factor, degranulation activity, cytotoxic activity, and cytokines secreted by NK cell stimulation.

7. The method of claim 6, wherein the cytokines are selected from IFN-_{Y}, TNF-α, TNF-β, MIP-1α, MIP-113, PANTES, IL-8, and IL-10.

8. Use of a kit for diagnosis of hemophagocytic lymphohistiocytosis (HLH) or cancer in a sample, the kit comprising: at least one selected from an antibody or fragment thereof specific to each or all of two distinguishable factors on NK cells, a target cell or polypeptide that induces specific activity, a composition that specifically induces activity, and a reversible or irreversible agonist, as a stimulating material that stimulates two distinguishable factors on NK cells in a sample to thereby induce synergistic activity of the NK cells; and
a material for detecting the presence or absence of activation of the NK cells compared to normal NK cells,
wherein the two distinguishable factors are NKG2D and 2B4 (CD244).

## Patentansprüche

1. Verfahren zur Diagnose von hämophagozytischer Lymphohistiozytose (HLH) oder Krebs in einer Probe, wobei das Verfahren umfasst:
spezifisches Stimulieren von zwei unterscheidbaren Faktoren auf NK-Zellen in einer Probe;
Messen eines synergistischen Aktivierungsniveaus der NK-Zellen, das durch die Stimulation der beiden Faktoren induziert wird; und
Vergleichen des synergistischen Aktivierungsniveaus der NK-Zellen mit dem von normalen NK-Zellen, wobei die beiden unterscheidbaren Faktoren NKG2D und 2B4 (CD244) sind.

2. Verfahren gemäß Anspruch 1, wobei die Faktoren auf den Oberflächen der NK-Zellen oder in den NK-Zellen vorhanden sind.

3. Verfahren gemäß Anspruch 1, wobei die Stimulierung der Faktoren gleichzeitig oder nacheinander durchgeführt wird.

4. Verfahren gemäß Anspruch 1, wobei die Stimulierung durch mindestens eines der Folgenden erfolgt: einen Antikörper oder ein Fragment davon, der/das für jeden oder alle Faktoren spezifisch ist, eine Zielzelle oder ein Polypeptid, die/das spezifische Aktivität induziert, eine Verbindung, die spezifisch Aktivität induziert, und einen reversiblen oder irreversiblen Agonisten.

5. Verfahren gemäß Anspruch 4, wobei die Zielzelle, die spezifische Aktivität in den Faktoren induziert, mit mindestens einem der Folgenden beschichtet ist: einem Antikörper gegen die beiden unterscheidbaren Faktoren auf NK-Zellen, einem Fragment davon, und einer Kombination davon.

6. Verfahren gemäß Anspruch 1, wobei die Messung das Messen mindestens eines der Folgenden umfasst: ein Phosphorylierungs- oder Expressionsniveau eines Unterfaktors jedes Faktors, eine Degranulationsaktivität, eine zytotoxische Aktivität, und durch NK-Zellstimulation sezernierte Zytokine.

7. Verfahren gemäß Anspruch 6, wobei die Zytokine ausgewählt sind aus IFN-γ, TNF-α, TNF-ß, MIP-1α, MIP-1ß, PANTES, IL-8 und IL-10.

8. Verwendung eines Kits zur Diagnose von hämophagozytischer Lymphohistiozytose (HLH) oder Krebs in einer Probe, umfassend mindestens eines der Folgenden: einen Antikörper oder ein Fragment davon, der/das für jeden oder alle von zwei unterscheidbaren Faktoren auf NK-Zellen spezifisch ist, eine Zielzelle oder ein Polypeptid, das spezifische Aktivität induziert, eine Zusammensetzung, die spezifisch Aktivität induziert, und einen reversiblen oder irreversiblen Agonisten, als stimulierendes Material, das zwei unterscheidbare Faktoren auf NK-Zellen in einer Probe stimuliert, um dadurch synergistische Aktivität der NK-Zellen zu induzieren; und
ein Material zum Nachweis des Vorhandenseins oder der Abwesenheit einer Aktivierung der NK-Zellen im Vergleich zu normalen NK-Zellen,
wobei die beiden unterscheidbaren Faktoren NKG2D und 2B4 (CD244) sind.

## Revendications

1. Procédé pour diagnostiquer une lymphohistiocytose hémophagocytaire (HLH)
ou un cancer dans un échantillon, le procédé consistant à :
stimuler spécifiquement deux facteurs différenciables sur des cellules tueuses naturelles dans un échantillon ;
mesurer un niveau d'activation synergique des cellules tueuses naturelles, provoqué par la stimulation des deux facteurs ; et
comparer le niveau d'activation synergique des cellules tueuses naturelles à celui de cellules tueuses naturelles normales
dans lequel les deux facteurs différenciables sont NKG2D et 2B4 (CD244).

2. Procédé selon la revendication 1, dans lequel les facteurs sont présents sur des surfaces des cellules tueuses naturelles ou dans les cellules tueuses naturelles.

3. Procédé selon la revendication 1, dans lequel la stimulation est effectuée sur les facteurs de manière simultanée ou séquentielle.

4. Procédé selon la revendication 1, dans lequel la stimulation est effectuée par au moins l'un sélectionné parmi un anticorps ou un fragment de celui-ci spécifique à chacun ou à la totalité des facteurs, une cellule ou un polypeptide cible qui provoque une activité spécifique, un composé qui provoque spécifiquement l'activité, et un agoniste réversible ou irréversible.

5. Procédé selon la revendication 4, dans lequel la cellule cible qui provoque l'activité spécifique dans les facteurs est recouverte d'au moins un sélectionné parmi un anticorps contre les deux facteurs différenciables sur les cellules tueuses naturelles, un fragment de celui-ci, et une combinaison de ceux-ci.

6. Procédé selon la revendication 1, dans lequel la mesure comprend la mesure d'au moins un sélectionné parmi un niveau de phosphorylation ou d'expression d'un sous-facteur de chaque facteur, une activité de dégranulation, une activité cytotoxique, et des cytokines sécrétées par la stimulation des cellules tueuses naturelles.

7. Procédé selon la revendication 6, dans lequel les cytokines sont sélectionnées parmi IFN-_{Y}, TNF-α, TNF-β, MIP-1α, MIP-1β, PANTES, IL-8 et IL-10.

8. Utilisation d'un kit de diagnostic d'une lymphohistiocytose hémophagocytaire (HLH) ou d'un cancer dans un échantillon, le kit comprenant : au moins un sélectionné parmi un anticorps ou un fragment de celui-ci spécifique à chacun ou à la totalité de deux facteurs différenciables sur les cellules tueuses naturelles, une cellule ou un polypeptide cible qui provoque une activité spécifique, une composition qui provoque spécifiquement l'activité, et un agoniste réversible ou irréversible, en tant que matériau de stimulation qui stimule deux facteurs différenciables sur les cellules tueuses naturelles dans un échantillon afin de provoquer ainsi l'activité synergique des cellules tueuses naturelles ; et
un matériau pour détecter la présence ou l'absence d'activation des cellules tueuses naturelles par rapport à des cellules tueuses naturelles normales,
dans laquelle les deux facteurs différenciables sont NKG2D et 2B4 (CD244).
